# EUROPÄISCHE PATENTSCHRIFT

(11) **EP 0 434 959 B1**
(45) Veröffentlichungstag und Bekanntmachung des Hinweises auf die Patenterteilung: **18.01.1995**
(21) Anmeldenummer: 90121842.0
(22) Anmeldetag: 15.11.1990
(51) Int. Cl.: C07C 7/08, C07C 15/04, C07C 15/06

(54) **Verfahren zur gleichzeitigen Gewinnung von reinem Benzol und reinem Toluol**
Process for simultaneously obtaining purified benzene and purified toluene
Procédé d'obtention simultanée de benzène purifié et de toluène purifié

(30) Priorität: 23.12.1989 DE 3942950
(43) Veröffentlichungstag der Anmeldung: 03.07.1991
(73) Patentinhaber: Krupp Koppers GmbH, 45143 Essen (DE)
(72) Erfinder: Klaumünzner, Udo, W-4330 Mülheim/Ruhr (DE); Vollmer, Hans-Jürgen, Dr., W-4300 Essen (DE)

(56) Entgegenhaltungen:
- EP-A- 0 085 572
- DE-A- 1 543 119
- DE-A- 1 568 940

## Beschreibung

Die Erfindung betrifft ein Verfahren zur gleichzeitigen Gewinnung von reinem Benzol und reinem Toluol aus diese Aromaten enthaltenden Kohlenwasserstoffgemischen durch Extraktivdestillation mit N-Formylmorpholin und/oder anderen N-substituierten Morpholinen, deren Substituenten nicht mehr als sieben C-Atome enthaltend, als selektivem Lösungsmittels, wobei das Einsatzprodukt vor der Einleitung in die Extraktivdestillationskolonne einer Vordestillation unterworfen wird, in der die höher als die genannten Aromaten siedenden Bestandteile als Sumpfprodukt abgetrennt werden.

Die Abtrennung von Aromaten aus Kohlenwasserstoffgemischen durch Extraktivdestillation mit den genannten N-substituierten Morpholinen als selektivem Lösungsmittel ist bereits seit längerer Zeit aus der DE-PS 15 68 940 bekannt. Unter Verwendung von N-Formylmorpholin als selektivem Lösungsmittel hat sich die dort beschriebene Arbeitsweise in der Praxis in zahlreichen großtechnischen Anlagen bewährt. Sofern hierbei das Einsatzprodukt neben Benzol noch größere Mengen an Toluol sowie gegebenenfalls Xylole enthält, ist es allerdings nicht zweckmäßig, dieses Einsatzprodukt unmittelbar einer Extraktivdestillation zu unterwerfen. Wegen der höheren Siedepunkte des Toluols und der Xylole muß nämlich in diesem Falle die Sumpftemperatur in der Extraktivdestillationskolonne soweit angehoben werden, daß auch die im Siedebereich dieser Aromaten siedenden Nichtaromaten vollständig aus dem Sumpf der Extraktivdestillation ausgedampft werden. Diese Erhöhung der Sumpftemperatur hat jedoch zur Folge, daß je nach Höhe der Temperatur eine mehr oder minder große Menge an Benzol zusammen mit den Nichtaromaten dampfförmig über Kopf aus der Extraktivdestillationskolonne entweicht, wodurch natürlich die Benzolausbeute entsprechend geringer wird.

Um dies zu vermeiden, ist deshalb in der DE-OS 15 43 119 ohne Bindung an ein spezifisches Lösungsmittel vorgeschlagen worden, das Einsatzprodukt vor der Einleitung in die Extraktivdestillationskolonne einer Vordestillation zu unterwerfen, bei der die höher als die zu gewinnenden Aromaten siedenden Bestandteile als Sumpfprodukt abgetrennt werden, während das dabei anfallende, die Aromaten enthaltende Kopfprodukt ohne weitere Auftrennung in die Extraktivdestillationskolonne eingeleitet wird. Das heißt, bei diesem Verfahren werden je nach Einsatzprodukt und Verfahrensführung entweder Benzol und Toluol oder Benzol, Toluol und Xylol gemeinsam mit den entsprechend siedenden Nichtaromaten in die Extraktivdestillationskolonne eingeleitet.

Diese Arbeitsweise, die in der Praxis auch mit den weiter oben genannten Lösungsmitteln angewandt wird, führt bei der gleichzeitigen Gewinnung von Benzol und Toluol aus einem diese Verbindungen enthaltenden Kopfprodukt jedoch zu dem Ergebnis, daß die Extraktivdestillation zwar ein hochreines Benzol liefert. Der Nichtaromatengehalt des gewonnenen Toluols liegt aber zum Teil noch bei ca.1,5 Gew.-%. Da jedoch für bestimmte Anwendungsfälle eine höhere Reinheit des Toluols erforderlich ist, kann dieses Ergebnis nur zum Teil befriedigen.

Der Erfindung liegt deshalb die Aufgabe zugrunde, das Verfahren der eingangs genannten Art dahingehend zu verbessern, daß die Reinheit des gewonnenen Toluols erhöht wird, ohne daß dies zu einer spürbaren Erhöhung der Anlage- und Betriebskosten führt.

Dies wird erfindungsgemäß dadurch erreicht, daß für die Extraktivdestillation eine Kolonne verwendet wird, die durch einen im mittleren Bereich angeordneten Kaminboden in ein Ober- und ein Unterteil getrennt ist und daß aus dem Einsatzprodukt in der Vordestillation in eine im Bereich zwischen 75 und 85°C siedende Benzolfraktion sowie eine im Bereich zwischen 99 und 111 °C siedende Toluolfraktion abgetrennt wird, wobei die Benzolfraktion in der Mitte des Unterteiles, die Toluolfraktion in der Mitte des Oberteiles und das Lösungsmittel in zwei Teilströmen am Kopf der Kolonne sowie dicht unterhalb des Kaminbodens in die Extraktivdestillationskolonne eingeleitet werden.

Das heißt, bei der Durchführung des erfindungsgemäßen Verfahrens erfolgt im Gegensatz zu der bisher üblichen Arbeitsweise, bei der Benzol und Toluol gemeinsam als Kopfprodukt aus der Vordestillation abgezogen und in die Extraktivdestillationskolonne eingeleitet wurden, zunächst in der Vordestillation eine Auftrennung in eine Benzol- und eine Toluolfraktion, die dann getrennt voneinander an unterschiedlichen Stellen in die Extraktivdestillationskolonne eingeleitet werden. Diese ist zu diesem Zweck durch einen im mittleren Bereich der Kolonne angeordneten Kaminboden in ein Oberteil und ein Unterteil getrennt, die beide an ihrem oberen Ende mit Anschlüssen für die Aufgabe des Lösungsmittels versehen sind. Vorteilhafterweise weist dabei das Oberteil der Extraktivdestillationskolonne eine zusätzliche Einrichtung für die Wärmezufuhr auf, beispielsweise in Form eines in Höhe des Kaminbodens an der Extraktivdestillationskolonne angeordneten Umlaufkochers, durch den ein Teil der sich auf dem Kaminboden sammelnden Flüssigkeit geleitet und erhitzt wird.

Weitere Einzelheiten des erfindungsgemäßen Verfahrens ergeben sich aus den vorliegenden Unteransprüchen und sollen nachfolgend an Hand der Abbildungen erläutert werden. Hierbei zeigen:
- Fig. 1: ein Fließschema der bisher üblichen Arbeitsweise
- Fig. 2: ein Fließschema des erfindungsgemäßen Verfahrens
Die dargestellten Fließschemata zeigen dabei nur die für die Verfahrenserläuterung unbedingt notwendigen Einrichtungen, während sonstige Nebeneinrichtungen, wie z.B. Umlaufkocher, Wärmetauscher, Ventile und Pumpen sowie die Einrichtungen für die Lösungsmittelregeneration nicht dargestellt sind.

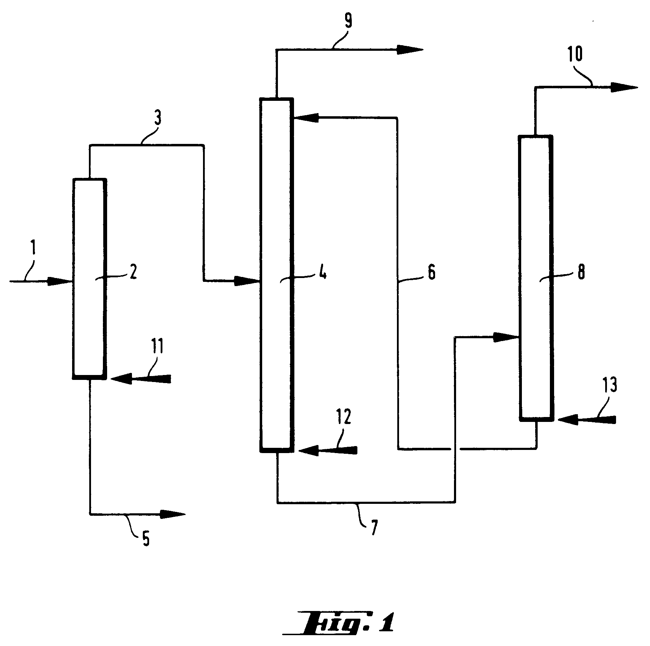

Bei dem Fließschema in Fig. 1 wird das Einsatzprodukt über die Leitung 1 in die Vordestillationskolonne 2 eingeleitet. Bei dem Einsatzprodukt kann es sich hierbei um unterschiedliche Benzol, Toluol und Xylole enthaltende Kohlenwasserstoffgemische, wie zum Beispiel Kokereibenzol-Druckraffinat, Pyrolysebenzin oder Reformatbenzin, handeln. In der Vordestillationskolonne 2, die mit Böden oder sonstigen Einbauten versehen sein kann, wurden bisher das Benzol und das Toluol gemeinsam mit den entsprechend siedenden Nichtaromaten über Kopf abdestilliert und über die Leitung 3 in den mittleren Teil der Extraktivdestillationskolonne 4 eingeleitet. Die höher siedenden Kohlenwasserstoffe wurden dagegen als Sumpfprodukt über die Leitung 5 aus der Vordestillationskolonne 2 abgezogen. In der Extraktivdestillationskolonne 4, die ebenfalls mit Böden oder sonstigen Einbauten versehen sein kann, erfolgte die Abtrennung der Nichtaromaten von den Aromaten, wobei das erforderliche Lösungsmittel (z.B.N-Formylmorpholin) über die Leitung 6 am Kopf auf die Extraktivdestillationskolonne 4 aufgegeben wird. Die Aromaten wurden dabei zusammen mit dem Lösungsmittel als Extrakt aus dem Sumpf der Extraktivdestillationskolonne abgezogen und gelangen über die Leitung 7 in die Abtriebskolonne 8, während gleichzeitig die Nichtaromaten dampfförmig über Kopf aus der Extraktivdestillationskolonne 4 entweichen und durch die Leitung 9 ihrer weiteren Behandlung zugeführt wurden. In der Abtriebskolonne 8 erfolgt die Abtrennung der Aromaten vom Lösungsmittel, wobei die Aromaten als Kopfprodukt über die Leitung 10 aus der Abtriebskolonne 8 abgezogen und anschließend in einer im Fließschema nicht dargestellten Kolonne destillativ voneinander getrennt wurden. Das von den Aromaten befreite Lösunsmittel wurde aus dem Sumpf der Abtriebskolonne 8 entfernt und über die Leitung 6 zwecks Wiederverwendung zur Extraktivdestillationskolonne 4 zurückgeführt. Die Pfeile 11, 12 und 13 markieren die Wärmezufuhr zu den einzelnen Kolonnen, die beispielsweise durch entsprechend angeordnete Umlaufkocher erfolgen kann.

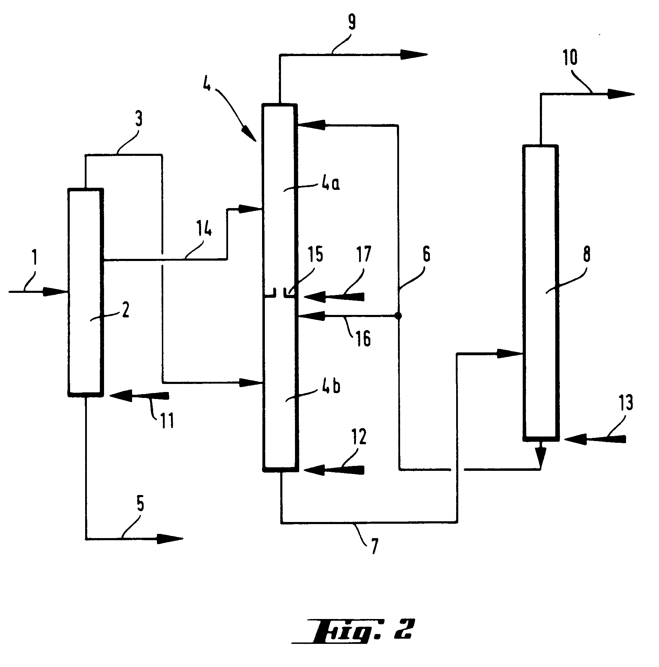

Wie bereits weiter oben festgestellt wurde, führt die vorstehend skizzierte Arbeitsweise zwar zu einem Benzol mit sehr hoher Reinheit, während die Reinheit des gewonnenen Toluols für bestimmte Anwendungsfälle nicht ausreichend ist. Da eine Nachreinigung des auf diese Weise gewonnenen Toluols auf wirtschaftliche Weise nicht möglich ist, stellt dies einen Nachteil dar, der nunmehr durch das erfindungsgemäße Verfahren, das in Fig. 2 dargestellt ist, aufgehoben wird. Der Aufbau der Anlage in Fig. 2 entspricht dabei prinzipiell dem Aufbau der Anlage in Fig. 1, wobei übereinstimmende Bezugszeichen selbstverständlich in beiden Figuren die gleiche Bedeutung haben. Die Vordestillationskolonne 2 wird hier jedoch unter solchen Bedingungen betrieben, daß über Kopf über die Leitung 3 nur die im Bereich zwischen 75 und 87°C siedende Benzolfraktion und über die die Leitung 14 als Seitenstrom die im Bereich zwischen 99 und 111°C siedende Toluolfraktion abgezogen wird, während die höher siedenden Bestandteile des Einsatzproduktes wie üblich über die Leitung 5 aus dem Sumpf der Vordestillationskolonne 2 entfernt werden. Die Extraktivdestillationskolonne 4 ist in diesem Falle durch den im mittleren Bereich dieser Kolonne angeordneten Kaminboden 15 in ein Oberteil 4a und ein Unterteil 4b getrennt. Bei einer Kolonne mit insgesamt 94 theoretischen Böden kann sich der Kaminboden 15 beispielsweise in Höhe des 53. Bodens von oben befinden. Die über die Leitung 3 abgezogene Benzolfraktion wird in der Mitte des Unterteiles 4b und die über die Leitung 14 abgezogene Toluolfraktion in der Mitte des Oberteiles 4a in die Extraktivdestillationskolonne 4 eingeleitet. Bei der vorstehend erwähnten Kolonne liegt beispielsweise die Einleitungsstelle für die Toluolfraktion in Höhe des 30. Bodens von oben und für die Benzolfraktion in Höhe des 77. Bodens von oben. Für den Oberteil 4a ist in diesem Falle eine zusätzliche Wärmezufuhr vorgesehen, was durch den Pfeil 17 angedeutet wird. Hierfür kann beispielsweise ein in Höhe des Kaminbodens 15 angeordneter Umlaufkocher verwendet werden. Prinzipiell entspricht die Arbeitsweise der Extraktivdestillationskolonne 4 auch in diesem Falle dem üblichen Schema. Das heißt, die Nichtaromaten werden als Raffinat über Kopf durch die Leitung 9 abgezogen, während die Aromaten zusammen mit dem Lösungsmittel als Extrakt aus dem Sumpf der Extraktivdestillationskolonne 4 abgezogen und über die Leitung 7 in die Abtriebskolonne 8 eingeleitet werden, in der die übliche Abtrennung der Aromaten vom Lösungsmittel erfolgt. Von dem über die Leitung 6 abgezogenen Lösungsmittel wird ein Teilstrom über die Leitung 16 abgezweigt und in den Unterteil 4b dicht unterhalb des Kaminbodens 15 eingeleitet, während das übrige Lösungsmittel über die Leitung 6 am Kopf in die Extraktivdestillationskolonne 4 eingeleitet wird. Bei der weiter oben erwähnten Kolonne mit 94 theoretischen Böden liegt die Eintrittsstelle für die Leitung 6 beispielsweise in Höhe des 1. Bodens von oben und für die Leitung 16 in Höhe des 54. Bodens von oben. Die Aufteilung des Lösungsmittels erfolgt dabei in der Weise, daß die Teilströme äquivalent zu den jeweiligen Einsatzproduktmengen sind. Bei der Durchführung des erfindungsgemäßen Verfahrens wird mit einem Verhältnis von Einsatzprodukt zu Lösungsmittel im Bereich von 1 : 3 bis 1 : 6 gearbeitet. Die Auftrennung des über die Leitung 10 abgezogenen Aromatenstromes führt zu einem Toluol, dessen Reinheit wesentlich besser ist, als dies bei Anwendung der zum Stande der Technik gehörenden Arbeitsweise möglich war.

Dies wird durch den nachfolgenden Vergleichsversuch belegt, bei dem das erfindungsgemäße Verfahren (Fig. 2) mit der zum Stande der Technik gehörenden Arbeitsweise (Fig. 1) verglichen wurde. Ausgehend von dem gleichen Einsatzprodukt und der gleichen Einsatzproduktmenge sowie dem gleichen Verhältnis von Einsatzprodukt zu Lösungsmittel (N-Formylmorpholin) von 1 : 4,3, wurden dabei die in der nachfolgenden Tabelle zusammengefaßten Versuchsergebnisse erzielt:

| | | Erfindung Fig.2 | Stand der Technik Fig.1 |
|---|---|---|---|
| Kopfprodukt Leitung 9 | (kg/h) | 9 342 | 9.109 |
| Benzol im Kopfprodukt | (%) | 4,81 | 12,32 |
| | (kg/h) | 449 | 1 123 |
| Toluol im Kopfprodukt | (%) | 5,78 | 1,73 |
| | (kg/h) | 540 | 158 |
| Benzolausbeute | (%) | 98,48 | 96,19 |
| Toluolausbeute | (%) | 98,33 | 49,51 |
| Nichtaromaten im Benzol | (ppm) | 931 | 4 |
| Nichtaromaten im Toluol | (ppm) | 2 020 | 18.130 |
| Wärme-Menge | (MW) | 20,05 | 20,05 |

Der Vergleichsversuch zeigt, daß bei Anwendung des erfindungsgemäßen Verfahrens die Reinheit des gewonnenen Toluols ganz erheblich verbessert werden kann. Zwar ist im Vergleichsversuch die Reinheit des gewonnenen Benzols bei Anwendung des erfindungsgemäßen Verfahrens gegenüber der bekannten Arbeitsweise etwas zurückgefallen, dafür lag die, Benzolausbeute jedoch höher. Werden die Verfahrensbedingungen beim erfindungsgemäßen Verfahren aber so eingestellt, daß die Benzolausbeute entsprechend verringert wird, so lassen sich bei ihm ebenfalls ähnlich hohe Benzolreinheiten erzielen, wie dies beim Verfahren nach dem Stande der Technik möglich ist. Da der Energiebedarf bei beiden Verfahren gleich groß ist, kann die der Erfindung zugrundeliegende Aufgabe als vollständig gelöst angesehen werden. Ein Vergleich der Fließschemata in Fig. 1 und 2 zeigt darüber hinaus ganz eindeutig, daß zur Durchführung des erfindungsgemäßen Verfahens nur kleinere Modifikationen an der Anlage gemäß Fig. 1 erforderlich sind. Das erfindungsgemäße Verfahren eignet sich deshalb auch zur Umrüstung bereits bestehender Anlagen, ohne daß hierfür erhebliche Investitionskosten erforderlich sind.

Abschließend soll noch darauf hingewiesen werden, daß die Vordestillation des Einsatzproduktes anstelle in einer Kolonne auch in zwei Kolonnen durchgeführt werden kann, wenn dies auf Grund der bestehenden betrieblichen Gegebenheiten zweckmäßiger ist.

## Patentansprüche

1. Verfahren zur gleichzeitigen Gewinnung von reinem Benzol und reinem Toluol aus diese Aromaten enthaltenden Kohlenwasserstoffgemischen durch Extraktivdestillation mit N-Formylmorpholin und/oder anderen N-substituierten Morpholine, deren Substituenten nicht mehr als sieben C-Atome enthalten, als selektivem Lösungsmittel, wobei das Einsatzprodukt vor der Einleitung in die Extraktivdestillationskolonne einer Vordestillation unterworfen wird, in der die höher als die genannten Aromaten siedenden Bestandteile als Sumpfprodukt abgetrennt werden, **dadurch gekennzeichnet**, daß für die Extraktivdestillation eine Kolonne verwendet wird, die durch einen im mittleren Bereich angeordneten Kaminboden in ein Ober- und ein Unterteil getrennt ist und daß aus dem Einsatzprodukt in der Vordestillation in eine im Bereich zwischen 75 und 85°C siedende Benzolfraktion sowie eine im Bereich zwischen 99 und 111 °C siedende Toluolfraktion abgetrennt wird, wobei die Benzolfraktion in der Mitte des Unterteiles die Toluolfraktion in der Mitte des Oberteiles und das Lösungsmittel in zwei Teilströmen am Kopf der Kolonne sowie dicht unterhalb des Kaminbodens in die Extraktivdestillationskolonne eingeleitet werden.

2. Verfahren nach Anspruch 1, **dadurch gekennzeichnet**, daß die für die Extraktivdestillation erforderliche Wärmezufuhr sowohl in den Unterteil als auch in den Oberteil der Extraktivdestillationskolonne erfolgt.

3. Verfahren nach den Ansprüchen 1 und 2, **dadurch gekennzeichnet**, daß die Extraktivdestillation mit einem Verhältnis von Einsatzprodukt zu Lösungsmittel im Bereich zwischen 1 : 3 und 1 : 6 durchgeführt wird.

## Claims

1. Process for the simultaneous isolation of pure benzene and pure toluene from hydrocarbon mixtures containing these aromatics by extractive distillation with, as selective solvent, N-formylmorpholine and/or other N-substituted morpholines whose substituents do not contain more than seven C atoms, the feedstock, before the introduction into the extractive distillation column, being subjected to a predistillation in which the constituents boiling higher than the said aromatics are separated off as bottom product, characterized in that for the extractive distillation, a column is used which is separated into an upper part and a lower part by a chimney-type tray arranged in the central region and in that from the feedstock in the predistillation separation is made into a benzene fraction boiling in the range between 75 and 85°C and into a toluene fraction boiling in the range between 99 and 111°C, the benzene fraction being introduced into the extractive distillation column in the centre of the upper part and the solvent being introduced into the extractive distillation column in two part-streams at the head of the column and closely beneath the chimney-type tray.

2. Process according to Claim 1, characterized in that the heat required for the extractive distillation is supplied both to the lower part and to the upper part of the extractive distillation column.

3. Process according to Claims 1 and 2, characterized in that the extractive distillation is carried out with a ratio of feedstock to solvent in the range between 1:3 and 1:6.

## Revendications

1. Procédé pour l'extraction simultanée de benzène pur et de toluène pur à partir de mélanges d'hydrocarbures contenant ces composés aromatiques par distillation d'extraction à l'aide de N-formylmorpholine et/ou d'autres morpholines N-substitutées, dont les substituants ne contiennent pas plus de sept atomes de C, en tant que solvant sélectif, le produit d'utilisation étant soumis avant introduction dans la colonne de distillation d'extraction à une distillation préalable, dans laquelle on sépare les composants à point d'ébullition plus élevé que celui desdits composés aromatiques, caractérisé en ce que l'on utilise pour la distillation d'extraction une colonne qui est séparée en une partie supérieure et en une partie inférieure par un fond de cheminée disposé dans le domaine médian, et que l'on sépare du produit d'utilisation dans la distillation préalable une fraction benzène à point d'ébullition dans le domaine entre 75 et 85°C ainsi qu'une fraction toluène à point d'ébulltion dans le domaine entre 99 et 111°C, la fraction benzène dans le milieu de la partie inférieure, la fraction toluène dans le milieu de la partie supérieure et le solvant étant introduits dans la colonne de distillation d'extraction sous la forme de deux courants partiels en tête de colonne ainsi que dans le voisinage immédiat en dessous du fond de cheminée.

2. Procédé selon la revendication 1, caractérisé en ce que l'apport de chaleur nécessaire pour la distillation d'extraction se fait tant dans la partie inférieure qu'aussi dans la partie supérieure de la colonne de distillation d'extraction.

3. Procédé selon les revendications 1 et 2, caractérisé en ce que l'on effectue la distillation d'extraction avec un rapport du produit d'utilisation au solvant dans le domaine entre 1 : 3 et 1 : 6.
